# EUROPEAN PATENT SPECIFICATION

(11) **EP 2 948 054 B1**
(45) Date of publication and mention of the grant of the patent: **06.06.2018**
(21) Application number: 14704234.5
(22) Date of filing: 23.01.2014
(51) Int. Cl.: A61B 5/0408, A61B 5/053, A61F 13/02

(54) **METHODS OF MANUFACTURING WIRELESS SENSOR PATCHES**
VERFAHREN ZUR HERSTELLUNG VON PFLASTERN MIT DRAHTLOSEN SENSOREN
PROCÉDÉS DE FABRICATION DE TIMBRES CAPTEURS SANS FIL

(30) Priority: 23.01.2013 US 201361755629 P; 23.01.2013 US 201361755623 P
(43) Date of publication of application: 02.12.2015
(73) Proprietor: Avery Dennison Corporation, Glendale, CA 91203 (US)
(72) Inventor: ADAMS, James, Mentor, OH 44060 (US); ARMIJO, Edward, A., Mentor, OH 44060 (US); HRUSKA, Ryan, Concord Township, OH 44077 (US); ONDERISIN, Michael, R., Concord Township, OH 44077 (US); SILVESTRO, David, Mentor, OH 44060 (US)
(74) Representative: Müller-Boré & Partner Patentanwälte PartG mbB
(86) International application number: PCT/US2014/012734
(87) International publication number: WO 2014/116816

(56) References cited:
- WO-A1-2010/085755
- WO-A2-2010/107913
- US-A- 4 738 674
- US-A- 5 790 036
- US-A- 6 149 614
- US-A1- 2002 180 605
- US-A1- 2005 065 489
- US-A1- 2010 056 881
- US-A1- 2011 144 470
- US-B1- 6 385 473

## Description

### Field of the Invention

The present invention relates to methods of manufacturing a plurality of wireless sensor patches.

### Background of the Invention

State of the art manufacturing methods of wireless sensor patches are for instance described in US patent publications US 5,790,036 and US 6,385,473.

It is known to sensors to monitor various parameters of a patient. Such sensors may include temperature sensors, Electrocardiogram (ECG) sensors, Galvanic Skin Response (GSR) sensors depending on the application of the sensor. In some applications, the sensors may be attached by wire to a device configured to process and/or display information obtained by the sensors. In further examples, sensors are known to comprise wireless sensors that communicate with another device wirelessly. There is a desire to provide wireless sensor patches to monitor various parameters of a patient. There is also a desire to provide manufacturing methods to sequentially manufacture a plurality of wireless sensor patches in an efficient and cost effective manner.

### Brief Summary of the Invention

The embodiments of the present invention described below are not intended to be exhaustive or to limit the invention to the precise forms disclosed in the following detailed description. Rather, the embodiments are chosen and described so that others skilled in the art may appreciate and understand the principles and practices of the present invention.

In a first aspect of the disclosure, a method of manufacturing a plurality of wireless sensor patches comprises the step (I) of unwinding a skin-friendly adhesive membrane from a skin-friendly adhesive membrane storage roll. The skin-friendly adhesive membrane includes a substrate sheet carrying a layer of skin-friendly adhesive and a first release liner carried by the layer of skin-friendly adhesive. The layer of skin-friendly adhesive is sandwiched between the first release liner and the substrate sheet. The method further comprises the step (II) of kiss cutting the substrate sheet and the skin-friendly adhesive to the first release liner to define a skin-friendly adhesive patch. The method still further includes the step (III) of unwinding a flexible support membrane from a support membrane storage roll, wherein the flexible support membrane includes a flexible support sheet with a skin adhesive layer applied to a first face of the flexible support sheet and a second release liner carried by the skin adhesive layer with the skin adhesive layer being sandwiched between the second release liner and the flexible support sheet. The method further includes the step (IV) of kiss cutting the flexible support sheet and the skin adhesive layer to the second release liner to define an opening extending through the flexible support sheet and the skin adhesive layer. The method still further includes the step (V) of removing the second release liner to expose the skin adhesive layer. The method also includes the step (VI) of laminating the substrate sheet to the flexible support sheet with the skin adhesive layer of the flexible support membrane. The method also includes the step (VII) of unwinding a tie layer membrane from a tie layer membrane storage roll, wherein the tie layer membrane includes a tie layer carrying a third release liner. The method further includes the step (VIII) of kiss cutting the tie layer to the third release liner to define a tie layer patch and the step (IX) of laminating the tie layer patch to the flexible support sheet. The method also includes the step (X) of cutting at least one probe opening through the tie layer membrane in alignment with the opening defined during step (IV) and the step (XI) of removing the third release liner to expose the tie layer patch laminated to the flexible support sheet. The method further includes the step (XII) of mounting a sensor device with respect to the flexible support membrane with a sensor probe in alignment with corresponding openings defined during steps (IV) and (X). The method also includes the step (XIII) of unwinding a flexible cover membrane from a flexible cover membrane storage roll and the step (XIV) of forming a pocket within the flexible cover membrane. The method further includes the step (XV) of cutting the flexible cover membrane to define a flexible cover patch including the pocket and the step (XVI) of laminating the flexible cover patch to the tie layer patch with the sensor device being at least partially received in the pocket. The method also includes the step (XVII) of cutting the flexible support sheet and the skin adhesive layer to provide a wireless sensor patch.

In one example of the first aspect, step (XVII) provides an outer periphery of the skin adhesive layer circumscribing the skin-friendly adhesive patch.

In another example of the first aspect, the skin-friendly adhesive comprises a hydrocolloid skin adhesive.

In another example of the first aspect, the flexible support membrane comprises a fabric, such as a nonwoven fabric.

In another example of the first aspect, step (XVII) is performed periodically to sequentially produce a plurality of wireless sensor patches.

In still another example of the first aspect, the method includes the step of providing indicia to a portion each wireless sensor patch containing information that matches information of indicia provided on a corresponding package housing each wireless sensor patch.

In still another example of the first aspect, the method further comprises the step of associating indicia of a portion of the wireless sensor patch with a batch of at least one source of assembly materials used to manufacture the wireless sensor patch.

The first aspect may be provided alone or in combination with any one or more of the examples of the first aspect discussed above.

Other features and advantages of the present invention will become apparent to those skilled in the art from the following detailed description. It is to be understood, however, that the detailed description of the various embodiments and specific examples, while indicating preferred and other embodiments of the present invention, are given by way of illustration and not limitation. Many changes and modifications within the scope of the present invention may be made without departing from it, and the invention includes all such modifications, as long as they fall under the scope of protection as defined by the claims.

### Brief Description of the Drawings

These, as well as other objects and advantages of this invention, will be more completely understood and appreciated by referring to the following more detailed description of the presently preferred exemplary embodiments of the invention in conjunction with the accompanying drawings, of which:
**FIG. 1** is example an example wireless sensor patch in accordance with aspects of the disclosure applied to a skin surface of a patient;
**FIG. 2** is a top view of the wireless sensor patch of **FIG. 1****;**
**FIG. 3** is a side view of the wireless sensor patch of **FIG. 1****;**
**FIG. 4** is a bottom view of the wireless sensor patch of **FIG. 1****;**
**FIG. 5** is an exploded upper perspective view of a wireless sensor patch of **FIG. 1****;**
**FIG. 6** is a sectional view of the wireless sensor patch along line 6-6 of **FIG. 5****;**
**FIG. 7** is a sectional view of the wireless sensor patch along line 7-7 of **FIG. 5****;**
**FIG. 8** is a bottom perspective view of a sensor device of the wireless sensor patch of **FIG. 5****;**
**FIG. 9** illustrates method steps in a method of manufacturing a plurality of wireless sensor patches;
**FIG. 10** is a sectional view along line 10-10 of **FIG. 9****;**
**FIG. 11** is a view along line 11-11 of **FIG. 9****;**
**FIG. 12** is a sectional view along line 12-12 of **FIG. 9****;**
**FIG. 13** is a view along line 13-13 of **FIG. 9****;**
**FIG. 14** is a view along line 14-14 of **FIG. 9****;**
**FIG. 15** is a continuation of **FIG. 9** illustrating further method steps in the method of manufacturing the plurality of wireless sensor patches;
**FIG. 16** is a sectional view along line 16-16 of **FIG. 15****;**
**FIG. 17** is a view along line 17-17 of **FIG. 15****;**
**FIG. 18** is a view along line 18-18 of **FIG. 15****;**
**FIG. 19** is a view along line 19-19 of **FIG. 15****;**
**FIG. 20** is a sectional view along line 20-20 of **FIG. 15****;**
**FIG. 21** is a sectional view along line 21-21 of **FIG. 15****;**
**FIG. 22** is a view along line 22-22 of **FIG. 15****;**
**FIG. 23** is a view along line 23-23 of **FIG. 15****;**
**FIG. 24** is a view along line 24-24 of **FIG. 15****;**
**FIG. 25** is a sectional view along line 25-25 of **FIG. 24****;**
**FIG. 26** is a continuation of **FIG. 15** illustrating further method steps in the method of manufacturing the plurality of wireless sensor patches;
**FIG. 27** is a view along line 27-27 of **FIG. 26****;**
**FIG. 28** is a view along line 28-28 of **FIG. 26****;**
**FIG. 29** is a view along line 29-29 of **FIG. 26****;**
**FIG. 30** is a continuation of **FIG. 26** illustrating further method steps in the method of manufacturing the plurality of wireless sensor patches;
**FIG. 31** is a view along line 31-31 of **FIG. 30****;**
**FIG. 32** is a sectional view along line 32-32 of **FIG. 31****;**
**FIG. 33** is cross-sectional view of another example wireless sensor patch in accordance with aspects of the disclosure;
**FIG. 34** is a bottom view of the wireless sensor patch of **FIG. 33** along line 34-34 of **FIG. 33****,** wherein the release liner is not illustrated for clarity;
**FIG. 35** is cross-sectional view of still another example wireless sensor patch in accordance with aspects of the disclosure;
**FIG. 36** is cross-sectional view of yet another example wireless sensor patch in accordance with aspects of the disclosure;
**FIG. 37** is a top view of the wireless sensor patch of **FIG. 36** along line 37-37 of **FIG. 36****;**
**FIG. 38** is cross-sectional view of another example wireless sensor patch in accordance with aspects of the disclosure; and
**FIG. 39** is a bottom view of the wireless sensor patch of **FIG. 38** along line 39-39 of **FIG. 38****,** wherein the release liner is not illustrated for clarity.

### Detailed Description of the Invention

The present invention is now illustrated in greater detail by way of the following detailed description which represents the best presently known mode of carrying out the invention. However, it should be understood that this description is not to be used to limit the present invention, but rather, is provided for the purpose of illustrating the general features of the invention.

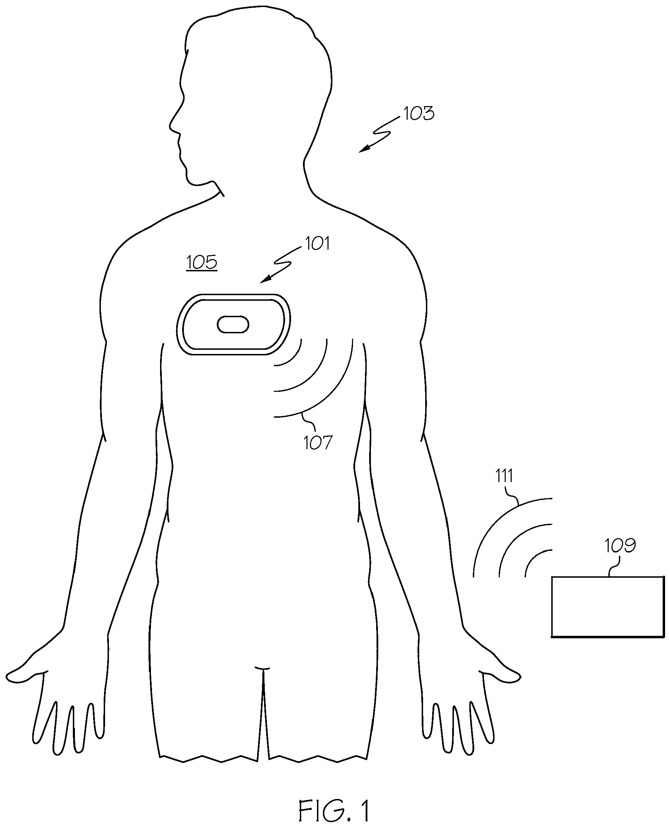

**FIG. 1** illustrates a wireless sensor patch **101** in accordance with aspects of the present disclosure. The wireless sensor patch **101** can be designed to monitor various parameters of a patient **103.** For instance, as shown, the wireless sensor patch **101** may be adhered to a skin surface **105** of the patient **103.** Any of the wireless sensor patches discussed throughout the disclosure may be adhered to various alternative skin surfaces of the patient. For instance, the skin patch may be adhered to the chest of a patient (e.g., as shown in **FIG. 1**), on the arm (e.g., on the back of the arm) of the patient or various other locations of the patient depending on the circumstances.

As discussed below, the wireless sensor patch **101** can include at least one sensor configured to monitor any one or combination of parameters. For instance, the sensor can comprise a Galvanic Skin Response (GSR) sensor configured to detect changes in the resistance of skin to electrical current due to changes in skin perspiration. Measuring the change in skin perspiration can be designed to detect various physiological and/or psychological conditions. In addition or alternatively, one or more Electrocardiogram (ECG) sensors may be provided to monitor the condition of the heart muscle in a patient. In still further examples, the sensors may be designed to detect features of the skin (e.g., temperature, glucose levels, levels of chemicals, pharmaceuticals, etc.). As such, the wireless sensor patch may have a wide range of applications. As discussed below, the wireless skin patch of the present disclosure can allow for comfortable application and monitoring without necessarily requiring a continuous wired connection. Moreover, the wireless sensor patch can be inexpensively produced, thereby rendering the patch potentially disposable. In disposable applications, a new wireless sensor patch to be provided for each application; thereby avoiding expensive sanitation and cleaning procedures.

The wireless sensor patch **101** may include a memory device configured to store data collected by the sensor device. In addition or alternatively, the wireless sensor patch **101** may include a transmitter configured to transmit wireless signals **107** (e.g., by way of Bluetooth wireless technology) to be received by a device **109.** The device **109** may be a cell phone or other receiving device that may in turn relay the information by satellite to another location for processing. In further examples, the wireless sensor patch may include a USB port or other interface to allow periodic wired connections with the wireless sensor patch. In such examples, after a period of time, the patient may temporarily provide a wired connection between the patch and the device **109** (e.g., by a USB cable) to provide communication between the device and the wireless sensor patch. In such a manner, information may be gathered by the device **109** continuously (e.g., by a wireless connection) in real time when synced with the device **109** and/or may be periodically sent to the device **109** when the patient makes a wired or other direct connection between the device **109** and the wireless patch. Such wired or wireless connections can be used to download information from the wireless sensor patch to the device **109** such as information gathered from the patient and/or current information about the wireless sensor patch. In further examples, the wired or wireless connection may allow information or commands to be uploaded from the device **109** to the wireless sensor patch. For instance commands may be uploaded to change an operating condition of the patch, to provide information to be displayed by the patch, and/or other functionality.

In further examples, the device **109** may comprise a storage unit configured to store data being transmitted by the wireless sensor patch **101**. In further examples, the device **109** may comprise a processing unit configured to process the data. In still further examples, the device **109** may optionally transmit signals **111** configured to be received by the wireless sensor patch **101.** For example, the device **109** may send command signals to the wireless sensor patch **101** to change an operating condition of the wireless sensor patch **101.**

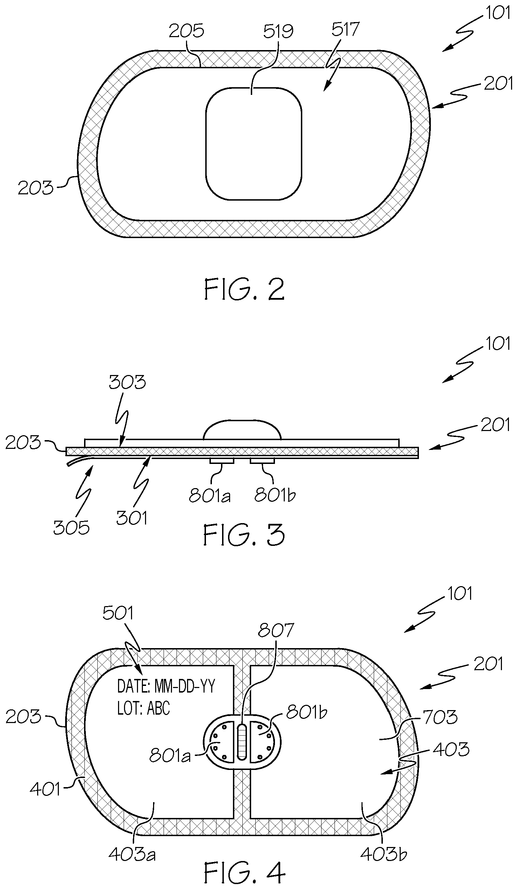

**FIG. 2** is a top view of the example wireless sensor patch **101** shown in **FIG. 1****.** The wireless sensor patch **101** includes a flexible support patch **201.** As shown in **FIG. 3****,** the flexible support patch **201** includes a thickness defined between a first face **301** and a second face **303.** As shown in **FIGS. 2 and 4****,** the flexible support patch **201** includes an outer periphery **203** defining a footprint of the flexible support patch **201.** The outer periphery **203** can comprise a wide range of shapes and sizes configured to be appropriately attached to the skin surface **105** of the patient **103.** The flexible support patch **201** can comprise a wide range of materials configured to provide support while still providing flexibility to allow the wireless sensor patch **101** to conform to a wide range of skin surface shapes. For example, the flexible support patch **201** can comprise a fabric a represented by the cross-hatch pattern illustrated in the drawings. The illustrated fabric comprises a nonwoven fabric although woven fabrics may be provided in further examples.

As represented by the vertical lines set forth in **FIG. 4****,** the wireless sensor patch **101** may also include an adhesive layer **401** applied to the first face **301** of the flexible support patch 201. The adhesive layer can comprise a pressure sensitive adhesive such as rubber-based adhesive, acrylic adhesive or silicone adhesive that allows the patch to immediately adhere to the skin surface **105** upon application of the wireless sensor patch **101**. Moreover, the wireless sensor patch **101** can comprise a skin-friendly adhesive patch **403** such as the illustrated first and second skin-friendly adhesive patch portions **403a, 403b** mounted to the first face **301** of the flexible support patch **201** with the adhesive layer **401.** In one example, the skin-friendly adhesive patch **403** can comprise a hydrocolloid adhesive patch although other skin-friendly adhesives may be provided such as integrated hydrocolloid or other adhesives capable of absorbing moisture. For example, the skin-friendly adhesive patch **403** can comprise a hydrocolloid such as the hydrocolloid material disclosed in any one of U.S. Patent No 7,335,416 that issued on February 6, 2008, U.S. Patent No 6,710,100 that issued on March 23, 2004, U.S. Patent No 6,583,220 that issued on June 24, 2003, U.S. Patent No 6,326,421 that issued on December 4, 2001, U.S. Patent Application No. 12/866,750 filed August 9, 2010, and U.S. Provisional Patent 61/467,553 filed March 25, 2011.

As shown in **FIG. 4****,** an outer periphery of the adhesive layer **401** can circumscribe the skin-friendly adhesive patch **403.** As such, an outer peripheral adherence of the wireless sensor patch **101** to the skin surface **105** of the patient **103** may be achieved. At the same time, the skin-friendly adhesive patch **403** may be held in place against the skin surface **105** to allow sufficient time for the skin-friendly adhesive patch **403** to cure into an effective adhesive member. The skin-friendly adhesive patch **403** allows the wireless sensor patch **101** to be applied to the skin surface for a significant length of time without aggravating the skin surface when compared to the adhesive layer **401.** At the same time, a relatively small peripheral portion of the adhesive layer **401** may allow the peripheral portions of the patch to be immediately adhered to the skin surface while allowing the skin-friendly adhesive patch **403** sufficient time to cure.

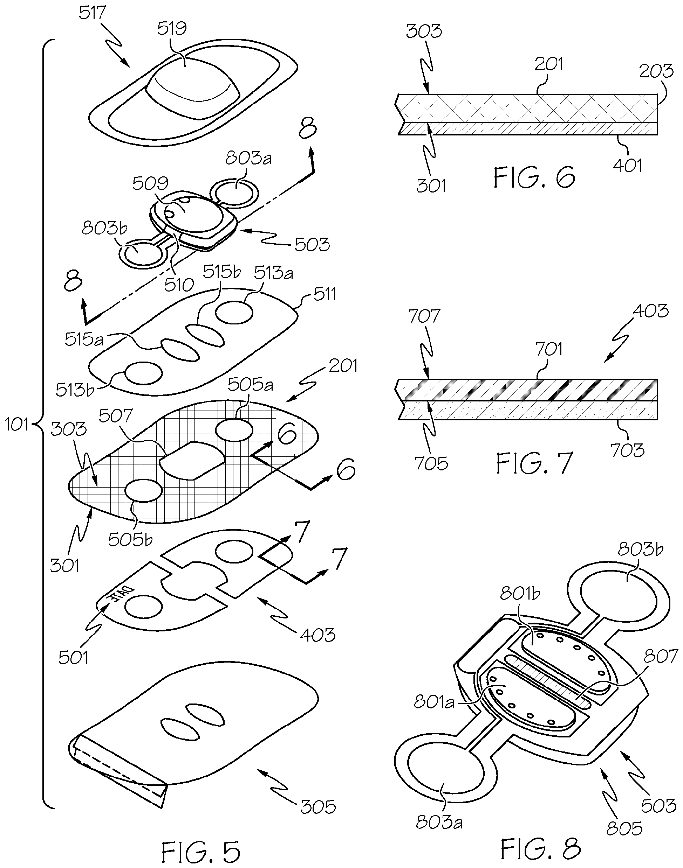

**FIG. 5** illustrates an exploded view of the wireless sensor patch **101.** **FIG. 6** is a cross-sectional view of the flexible support patch **201** dem onstrating the adhesive layer **401** b eing applied to the first face **301** of the flexible support patch **201.** **FIG. 7** is a cross-sectional view of the skin-friendly adhesive patch **403** along line 7-7 of **FIG. 5****.** As shown, the skin-friendly adhesive patch **403** can include a flexible substrate **701** with a skin-friendly adhesive layer **703** applied to a first face **705** of the flexible substrate **701.** In some examples, the flexible substrate **701** can comprise a transparent or translucent substrate **701** to allow viewing of indicia **501** that may be printed on the second face **707** of the flexible substrate (e.g., see **FIGS. 4** **and** **5**). In such examples, the indicia **501** may be printed in reverse such that the information may be deciphered by viewing the indicia through the skin-friendly adhesive layer and the flexible substrate **701.** The skin-friendly adhesive layer **703** can comprise a hydrocolloid adhesive or other skin-friendly adhesive that can facilitate adhesion to the skin surface for long periods of time relatively low aggravation to the skin layer.

As further illustrated in **FIG. 5****,** the wireless sensor patch **101** further includes a sensor device **503.** **FIG. 5** illustrates a top perspective view of one example sensor device **503** while **FIG. 8** illustrates a bottom perspective view of the sensor device **503** along line 8-8 of **FIG. 5****.** As shown in **FIG. 8****,** the sensor device **503** can include a pair of Galvanic Skin Response (GSR) sensor probes **801a**, **801b** configured to interact with the skin surface **105** to detect changes in the resistance of skin to electrical current due to changes in skin perspiration. The sensor device **503** can also include a pair of Electrocardiogram (ECG) sensor probes **803a**, **803b** configured to monitor the condition of the heart muscle in a patient.

An electronics module **805** may be provided that can receive signals from the pair of GSR sensor probes **801a, 801b** and/or the pair of ECG sensor probes **803a**, **803b**. In some examples, the wireless sensor patch **101** may only be configured to operate with one of the sensor types although both sensor types may be configured to operate in further examples. For instance, as shown in **FIG. 4****,** only the GSR sensor probes **801a, 801b** extend through the flexible support patch 201 to engage the skin surface of the patient. The ECG sensor probes **803a**, **803b** are not exposed to interact with the skin surface and/or the electronics within the electronic module **805** may be arranged to turn off the ECG sensor and/or may not have ECG sensor functionality. **FIG. 5** demonstrates an example where apertures **505a, 505b** are provided in the flexible support patch **201** in addition to a central aperture **507** to allow communication of the GSR sensor probes **801a**, **801b** and the ECG sensor probes **803a**, **803b** through the flexible support patch **201.**

As further shown in **FIG. 8****,** the sensor device **503** can also include identification indicia **807** such as a UPC code or the like to refer to the specific sensor device **503** or a type of sensor device. Referring back to **FIG. 5****,** the sensor device **503** can also include a battery **509** configured to power the sensor device **503** and a control button **510** configured to operate the sensor device. The sensor device **503** can be mounted to the second face **303** of the flexible support patch **201** such that at least one of the sensor probes is aligned with the apertures. Indeed, as shown, the GSR sensor probes **801a, 801b** are aligned with the central aperture **507** extending through the flexible support patch. Such alignment allows a portion of the GSR sensor probes **801a**, **801b** to protrude from the central aperture **507** as shown in **FIG. 3****.** Moreover, as shown in **FIG. 5****,** the ECG sensor probes **803a**, **803b** may be aligned with the corresponding apertures **505a, 505b** extending through the flexible support patch **201.** Although not shown, a hydrogel or conductive agent may also be provided to help achieve coupling of the ECG sensor probes **803a**, **803b** with the skin surface **105** through the corresponding apertures **505a, 505b**.

The sensor device **503** may be mounted to the second face **303** of the flexible support patch **201** with various adhesive configurations. For instance, as shown in **FIG. 5****,** adhesive in the form of a tie layer patch **511** can be provided to help mount the sensor device **503** to the second face **303** of the flexible support patch **201.** As shown, apertures **513a**, **513b** may be provided in alignment with the ECG sensor probes **803a**, **803b** to facilitate appropriate interaction with the skin surface **105.** Likewise, apertures **515a**, **515b** may be provided in alignment with the GSR sensor probes **801a, 801b** to facilitate appropriate interaction with the skin surface **105.** In some examples, the tie layer may be transparent and/or translucent to allow viewing of the identification indicia **807** from below as shown in **FIG. 4****.**

The wireless sensor patch **101** further includes a flexible cover patch **517** mounted to the second face **303** of the flexible support patch **201** wherein the sensor device **503** is at least partially housed within a pocket **519** defined by at least one of the flexible support patch **201** and the flexible cover patch **517.** For example, a protruding portion of the electronic module **805** can be housed within a preformed pocket **519** of the flexible cover patch **517.** In some designs, the control button **510** may be activated by depressing a side portion of the flexible preformed pocket **519.** The flexible cover patch may comprise a polymeric member, such as a closed cell foam material that may be substantially water resistant to help protect the electrical components of the electronic module **805.** As shown, the tie layer patch **511** may function to mount the flexible cover patch **517** to the second face 303 of the flexible support patch **201.**

As further illustrated in **FIG. 2****,** the flexible cover patch **517** includes an outer periphery **205** defining a footprint of the flexible cover patch **517.** As shown, in one example, the footprint of the flexible support patch **201** defined by the outer periphery **203** of the flexible support patch **201** is larger than a footprint of the flexible cover patch **517.** Providing the flexible cover patch **517** with a larger footprint can help prevent overlapping of the periphery **205** of the flexible cover patch **517** that may provide a peeling point. As such, the wireless sensor patch **101** can be securely applied to the skin surface **105** with a reduced chance of inadvertent peeling of the wireless sensor patch **101** from the skin surface **105.**

Referring to **FIGS. 3** **and** **5****,** a release liner **305** may be provided to help preserve the adhesive layer **401** and the skin-friendly adhesive layer **703** from adhering to other surfaces and/or contamination prior to application of the wireless sensor patch.

The wireless sensor patch **101** shown in **FIGS. 1-6** may be easily applied to the skin surface **105** of a patient **103.** As shown in **FIGS. 3** **and** **5****,** the release liner **305** may be initially removed to expose the adhesive layer **401** and the skin-friendly adhesive layer **703** as shown in **FIG. 4****.** Moreover, as shown in **FIG. 4****,** the indicia **501** may be read through the skin friendly adhesive. Moreover, the indicia **807** associated with the sensor device **503** may be read through the tie layer patch **511.** Next, the wireless sensor patch **101** may be applied to the skin surface **105** of a patient **103** at an appropriate location. Once applied, the outer peripheral portion of the adhesive layer **401** immediately mounts the wireless sensor patch **101** in place, wherein, after sufficient time, the skin-friendly adhesive layer **703** cures to provide the primary bonding while reducing irritation and/or aggravation to the skin layer that may otherwise occur over long periods of time with only the relatively harsh adhesive layer **401.**

Various methods may be used to manufacture the wireless sensor patch **101.** For example, referring to **FIG. 5****,** the method can include the step of providing the flexible support patch **201** including the first face **301** and the second face **303** with the outer periphery **203** defining the footprint of the flexible support patch **201.** The method can also include providing the adhesive layer **401** to the first face **301** of the flexible support patch **201.** In further examples, the skin-friendly adhesive patch **403** may be mounted to the first face **301 of** the flexible support patch **201** with the adhesive layer **401.** In one example, indicia **501** may be printed in reverse on the surface being adhered to the adhesive layer **401.** As such, the indicia may be viewed through the skin-friendly adhesive patch **403** as shown in **FIG. 4****.**

Referring back to **FIG. 5****,** the method of manufacturing the skin patch can further include the step of mounting the sensor device **503** to the second face **303** of the flexible support patch **201.** At least one sensor probe of the sensor device can be aligned with an aperture extending through the flexible support patch prior to mounting the sensor device to the second face of the flexible support patch. For example, as shown in **FIG. 5****,** the GSR sensor probes **801a, 801b** can be aligned with the central aperture **507** of the flexible support patch **201.** Likewise, the ECG sensor probes **803a, 803b** may be aligned with the corresponding apertures **505a, 505b** extending through the flexible support patch **201.**

The method of manufacturing can further include the step of mounting the flexible cover patch **517** to the second face **303** of the flexible support patch **201.** In one example, the tie layer patch **511** or other adhesive may be used to mount the flexible cover patch **517** to the second face **303** of the flexible support patch **201.** After mounting the flexible cover patch **517,** the protruding portion of the electronic module **805** is at least partially housed within the pocket **519** defined by the flexible cover patch **517.** Although not shown, in addition or alternatively, the flexible support patch **201** may include a pocket for at least a portion of the sensor device **503.**

The efficiency and speed of manufacturing the wireless sensor patch may be enhanced by various methods of manufacturing a plurality of wireless sensor patches, for example, sequentially manufacturing a plurality of the wireless sensor patches.

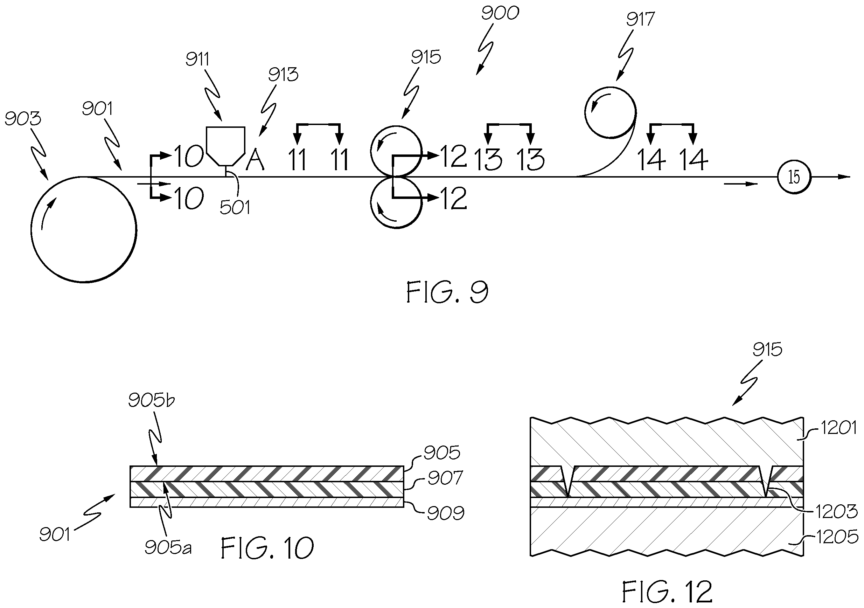

**FIG. 9** illustrates a schematic view of a manufacturing apparatus **900** demonstrating example method steps of manufacturing a plurality of wireless sensor patches. The method can include the step of unwinding a skin-friendly adhesive membrane 901 from a skin-friendly adhesive membrane storage roll **903.**

As shown in **FIG. 10****,** the skin-friendly adhesive membrane **901** can include a substrate sheet **905** carrying a layer of skin-friendly adhesive **907** on a first face **905a** of the substrate sheet **905.** The substrate sheet **905** will eventually be separated (e.g., cut) into the flexible substrate **701** of the skin-friendly adhesive patch **403** discussed above. As such, in some examples, the substrate sheet **905** can comprise a transparent or translucent substrate sheet to allow viewing of indicia **501** that may be subsequently printed on the second face **905b** of the substrate sheet **905.** In some examples, the substrate sheet **905** can comprise a sheet of flexible polyurethane that acts as a carrier sheet for the skin-friendly adhesive **907.**

As discussed previously, the skin-friendly adhesive membrane **901** further includes a layer of skin-friendly adhesive **907** that may be carried by the first face **905a** of the substrate sheet **905.** The skin-friendly adhesive layer **907** will eventually be processed into the skin-friendly adhesive layer **703** of the skin-friendly adhesive patch **403** discussed above. As such, the skin-friendly adhesive layer **907** can comprise a hydrocolloid adhesive or other skin-friendly adhesive that can facilitate adhesion to the skin surface for long periods of time relatively low damage to the skin layer. As further illustrated in **FIG. 10****,** the skin-friendly adhesive membrane **901** can further include a first release liner **909** carried by the layer of skin-friendly adhesive **907.** In one example, the first release liner **909** can comprise a siliconized release liner that can protect the skin-friendly adhesive **907** during transport while allowing easy removal of the release liner from contacting the skin-friendly adhesive **907** to expose the skin-friendly adhesive for further processing.

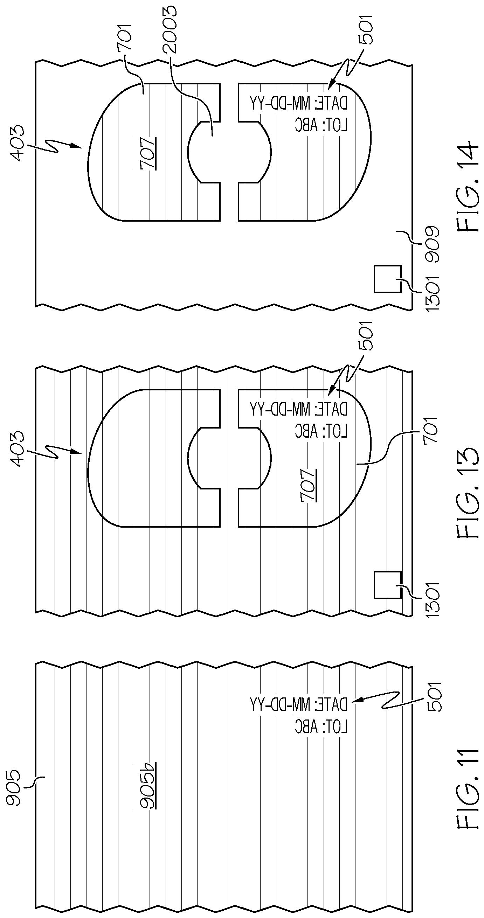

The manufacturing apparatus **900** can further include a printing device **911** configured to print indicia **501 on** the second face **905b** of the substrate sheet 905. As shown in **FIG. 11****,** the indicia may be printed in reverse such that the information may be deciphered by viewing the indicia through the skin-friendly adhesive layer and the flexible substrate through the first face **905a** of the substrate sheet **905.** The indicia may comprise information relating to the wireless sensor patch 101 such as the date of printing, the lot number and may even include identification information to allow individualized naming of each corresponding wireless sensor patch. The manufacturing apparatus **900** may also include a quality check, such as an optical reader **913** configured to check the printing quality of the indicia **501.**

The manufacturing apparatus **900** can also include a pair of rollers **915** configured to kiss cut the substrate sheet **905** and the skin-friendly adhesive **907** to the first release liner **909** to define the skin-friendly adhesive patch **403** shown in **FIG. 5****.** Indeed, as shown in **FIG. 12****,** one roller **1201** of the pair of rollers **915** can include a cutting knife **1203** in the shape of the skin-friendly adhesive patch **403.** As shown, the cutting knife **1203** is designed to work against an anvil roller **1205** to kiss cut the substrate sheet **905** and the skin-friendly adhesive **907** to the first release liner **909** without cutting through the release liner. As shown in **FIG. 13****,** the shape of the skin-friendly adhesive patch **403** is subsequently kiss cut within the skin-friendly adhesive membrane **901.** Once the kiss cut is complete, the indicia **501** may be app ropriately located on the second face **707** of the flexible substrate **701** of the skin-friendly adhesive patch **403.**

As further illustrated in **FIG. 13****,** a plurality of first registration openings **1301** may be provided by another knife of the pair of rollers (not shown). As shown, the first registration openings **1301** is created by a removed cut-out of the substrate sheet **905,** the skin-friendly adhesive **907** and the first release liner **909** of the skin-friendly adhesive membrane **901.** The cut-out may be provided periodically along the length of the skin-friendly adhesive membrane **901** (e.g., between each adjacent pair of kiss-cut skin-friendly adhesive patches **403** to facilitate registration of subsequent material layers as discussed more fully below.

Turning back to **FIG. 9****,** the manufacturing apparatus **900** may further include a take up roll **917** configured to wind up unused portions of the skin-friendly adhesive membrane **901** from the first release liner **909.** As shown in **FIG. 14****,** the kiss-cut skin-friendly adhesive patches **403** are thereafter left behind on the first release liner **909.**

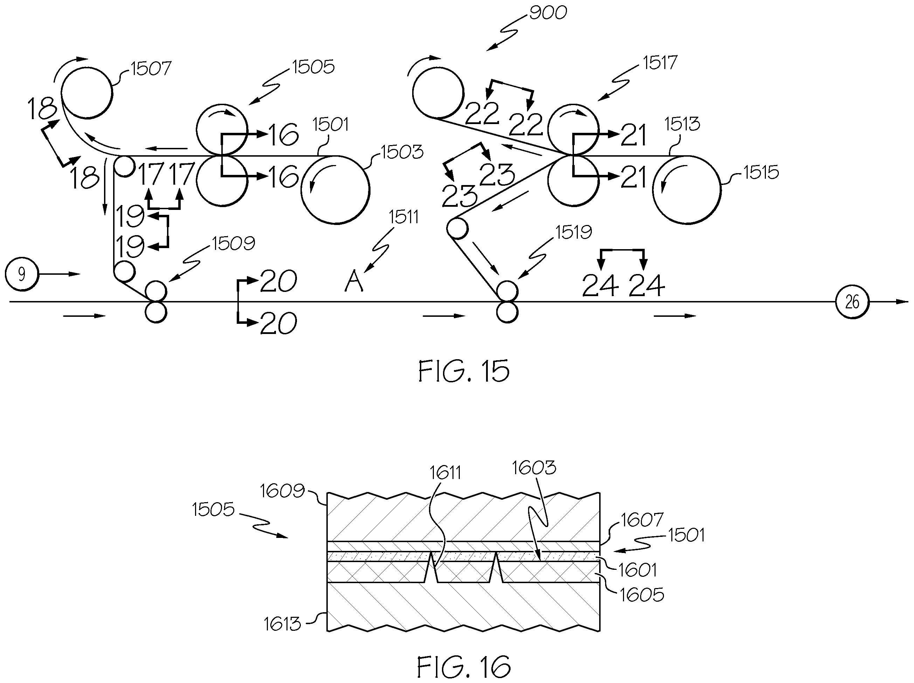

**FIG. 15** is a continuation of **FIG. 9** illustrating further method steps. The method further includes the step of unwinding a flexible support membrane **1501** from a support membrane storage roll **1503** along an assembly path. As shown in **FIG. 16****,** the flexible support membrane **1501** is provided with a skin adhesive layer **1601** applied to a first face **1603** of a flexible support sheet **1605.** The skin adhesive layer **1601** may eventually be cut into the skin adhesive layer **401** of the flexible support patch **201** shown in **FIG. 5** and/or **FIG. 6****.** As such, the skin adhesive **1601** may comprise a contact adhesive that may quickly mount the flexible support patch **201** to a skin surface **105.** The skin adhesive layer **1601** is therefore configured to adhere to a first face **1603** of the flexible support sheet **1605** such that the skin adhesive may be configured to adhere the first face **1603** of the flexible support sheet **1605** to a skin surface **105.** The flexible support sheet **1605** may be eventually cut into the flexible support patch **201.** As such, the flexible support sheet **1605** may comprise a fabric, such as a nonwoven fabric although other materials may be used in further examples.

As further illustrated in **FIG. 16****,** the flexible support membrane **1501** further includes a second release liner **1607** carried by the skin adhesive layer **1601** with the skin adhesive layer **1601** being sandwiched between the second release liner **1607** and the flexible support sheet **1605.** The second release liner **1607** may comprise a siliconized liner or other release liner configured to preserve the skin adhesive layer **1601** while further processing techniques are carried out as discussed more fully below.

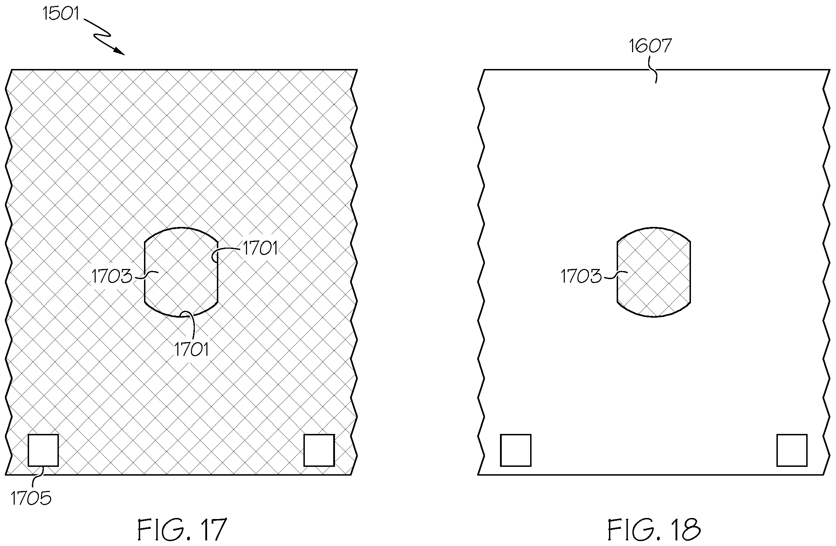

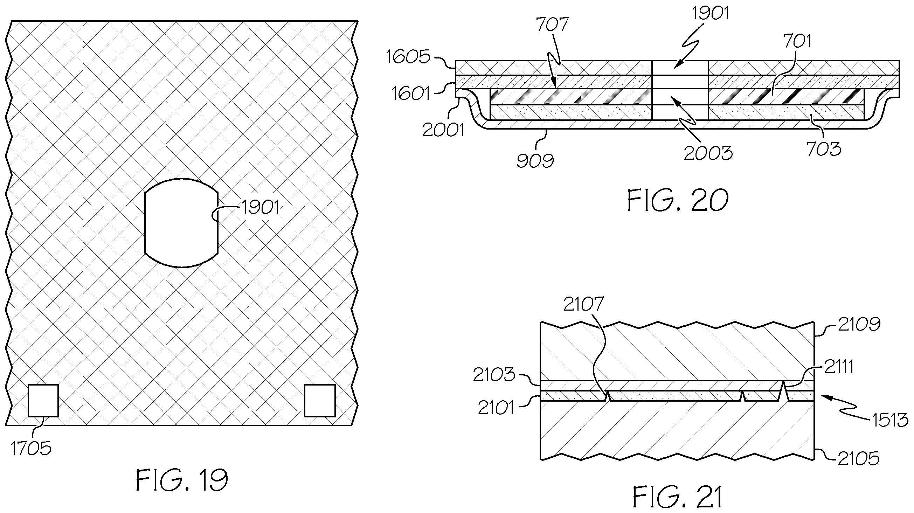

The method further includes the step of kiss cutting the flexible support sheet **1605** and the skin adhesive layer **1601** to the second release liner **1607** to define an opening **1901** (see **FIG. 19**) extending through the flexible support sheet **1605** and the skin adhesive layer **1601.** For example, as shown in **FIG. 15****,** the manufacturing apparatus **900** can also include a pair of rollers **1505** configured to kiss cut the flexible support sheet **1605** and the skin adhesive layer **1601** to the second release liner **1607.** As shown in **FIG. 16****,** one roller **1613** of the pair of rollers **1505** can include a cutting knife **1611** in the shape of the opening **1901.** As shown, the cutting knife **1611** is designed to work against an anvil roller **1609** to kiss cut the flexible support sheet **1605** and the skin adhesive layer **1601** to the second release liner **1607** without cutting through the second release liner. Once complete, as shown in **FIG. 17****,** a peripheral cut **1701** circumscribes a central tab **1703** in the shape of the opening **1901.**

Further, as illustrated in **FIG. 17****,** a plurality of second registration openings **1705** may be provided by another knife (not shown) of the pair of rollers **1505.** As shown in **FIG. 17****,** the second registration openings **1705** are created by a removed cut-out of the flexible support membrane **1501.** The cut-out may be provided periodically along the length of the flexible support membrane to confirm registration between the material layers as discussed more fully below.

Turning back to **FIG. 15****,** the manufacturing apparatus **900** may further include a take up roll **1507** configured to wind up the second release liner **1607** with the central tab **1703** being carried away with the second release liner **1607** as shown in **FIG. 18****.** As such, the second release liner **1607** is removed to expose the skin adhesive layer **601.** As shown in **FIG. 19****,** the flexible support sheet **1605** is then carried in a path toward the first release liner **909** wherein the skin adhesive layer **601** is laminated by rollers **1509** to the second face **707** of the flexible substrate **701** of the skin-friendly adhesive patch **403.**

**FIG. 20** is a sectional view of the flexible support sheet **1605** being mounted to the second face **707** of the flexible substrate **701** of the skin-friendly adhesive patch **403** by way of the skin adhesive layer **1601.** As with the other figures, the thicknesses of the layers in **FIG. 20** are exaggerated for clarity. As further illustrated in **FIG. 20****,** the outer portions **2001** of the first release liner **909** are temporarily attached to the skin adhesive layer **1601.** Once mounted, the opening **1901** of the flexible support sheet **1605** is aligned with the opening **2003** defined within a central portion of the skin friendly adhesive patch (see **FIG. 14**).

Turning back to **FIG. 15****,** a sensor **1511** such as the optical sensor can monitor the process to ensure proper alignment between the flexible support sheet **1605** and the first release liner **909.** If proper registration is achieved, the plurality of first registration openings **1301** are aligned with the plurality of second registration openings **1705.** The sensor **1511** can determine appropriate alignment of each corresponding pair of the registration openings **1301, 1705** to confirm appropriate registration and mounting of the flexible support sheet **1605** relative to the skin-friendly adhesive patch **403.**

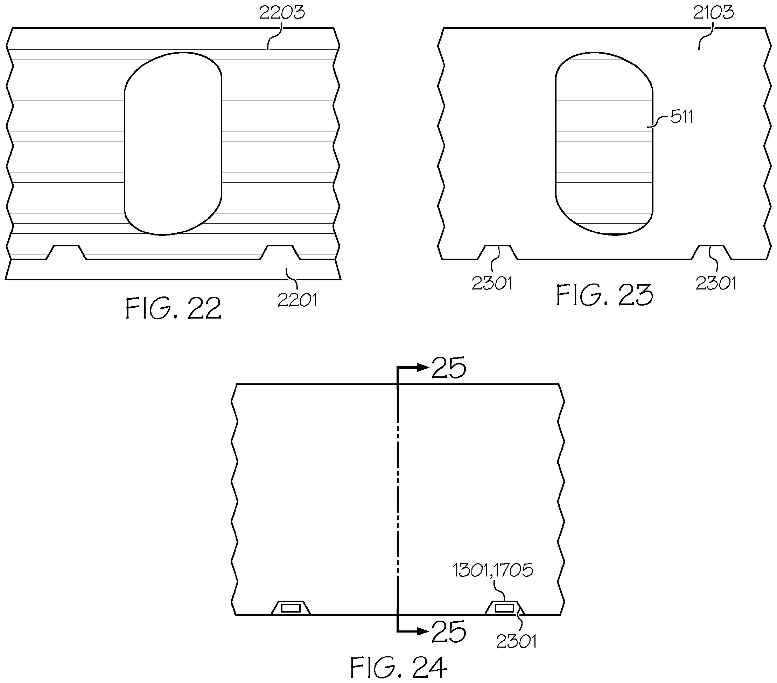

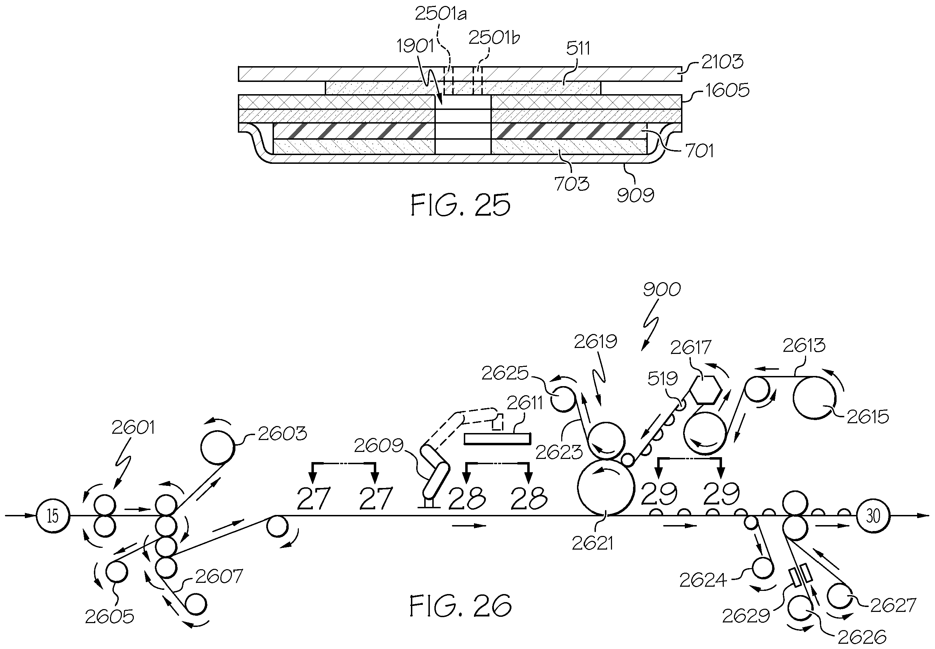

As shown in **FIG. 15****,** the manufacturing apparatus **900** further provides for the step of unwinding a tie layer membrane **1513** from a tie layer membrane storage roll **1515.** As shown in **FIG. 21****,** the tie layer membrane **1513** includes a tie layer **2101** carrying a third release liner **2103.** As further shown in **FIGS. 15** **and** **21****,** the manufacturing apparatus **900** also includes a pair of rollers **1517** configured to kiss cut the tie layer **2101** to the third release liner **2103** to define the tie layer patch **511** shown in **FIG. 5****.** As shown in **FIG. 21****,** one roller **2105** of the pair of rollers **1517** can include a cutting knife **2107** in the shape of the outer periphery of the tie layer patch **511.** The cutting knife **2107** is designed to work against an anvil roller **2109** to kiss cut the tie layer **2101** to the third release liner **2103** without cutting through the third release liner. At the same time, an edge die cut knife **2111** can trim an edge **2201** of the third release liner **2103** to follow with unused portions of the tie layer as shown in **FIG. 22****.** As shown in **FIG. 23****,** the remainder of the third release liner **2103** carries the tie layer patch **511** cut from the tie layer **2103** of the tie layer membrane **1513.** The third release liner **2103** carries the tie layer patch **511** to be laminated by rollers **1519** to the flexible support sheet **1605** as shown in **FIGS. 24** **and** **25****.** The remainder of the third release liner **2103** can include periodic cut outs **2301** that can provide an alignment check as shown in **FIG. 24** (e.g., by an optical sensor or the like).

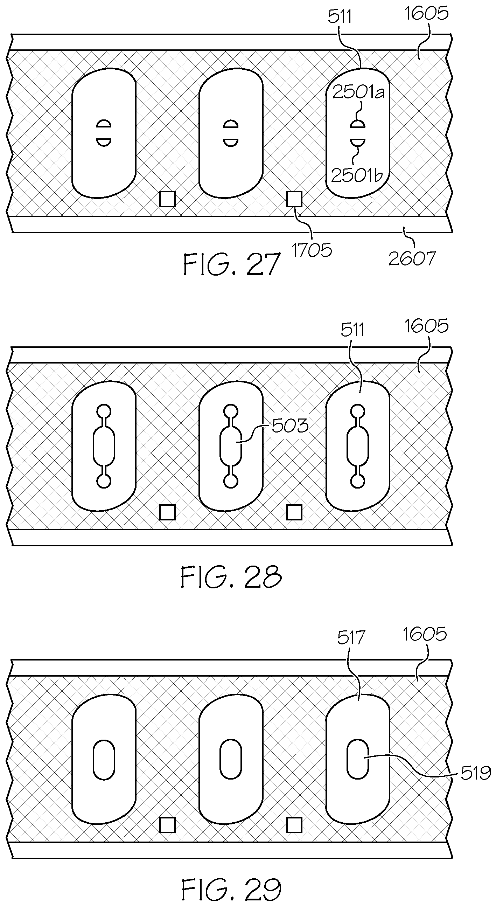

**FIG. 26** is a continuation of **FIG. 15** illustrating further method steps in the method of manufacturing the plurality of wireless sensor patches **101.** **FIG. 26** illustrates a pair of rollers **2601** configured to die cut at least one probe opening through the tie layer membrane in alignment with the opening **1901.** For example, as shown in dashed lines in **FIG. 25****,** the pair of rollers **2601** may cut GSR probe openings **2501a, 2501b** through the third release liner **2103** and the die layer patch **511.** The method then includes the step of removing the third release liner **2103** for being stored on take up roller **2603.** Once the third release liner **2103** is removed, the tie layer patch **511** laminated to the flexible support sheet **1605** is exposed. Next, the first release liner **909** can be removed and stored on take up roller **2605.** In place of the first release liner **909,** a clear support substrate **2607** may be applied to the skin friendly adhesive layer **907.** The result top view is shown in **FIG. 27** wherein the tie layer patches **511** are spaced apart in series form one another with GSR probe openings **2501a, 2501b** extending through the tie layer patches **511.**

Turning back to **FIG. 26****,** a robot **2609** picks one of a plurality of sensor devices **503** from a source of sensor devices **2611** and mount each sensor device **503** with respect to the flexible support sheet **1605** with the GSR probes **801a, 801b** extending through respective GSR probe openings **2501a, 2501b** within the tie layer patch **511.** A sensing device, such as a camera may view the sensor device **503** prior to placement to achieve proper positioning of the sensor device **503** as shown in **FIG. 28****.**

The flexible cover patch **517** may then be created and properly positioned over the tie layer patch **511** as discussed with reference to **FIGS. 26** **and** **29****.** For example, as shown in **FIG. 26****,** the method includes the step of unwinding a flexible cover membrane **2613** from a flexible cover membrane storage roll **2615.** The flexible cover membrane **2613** may be heated and then a vacuum formed pocket is generated by way of vacuum forming cylinder **2617** that includes a plurality of cavities that may suction the heated flexible cover membrane **2613** to form the pockets **519** in the flexible cover membrane **2613.** A pair of die cutting rollers **2619** then cut the flexible cover patches **517** from the flexible cover membrane **2613.** Each cut flexible cover patch **517** includes a corresponding one of the pockets **519.** Suction within a transfer cylinder **2621** transports the flexible cover patches **517** to be placed in alignment over a corresponding tie layer patch **511.** In fact, as shown in **FIG. 29****,** the footprint of the flexible cover patch **517** maybe substantially the same or slightly greater than or less than the footprint of the tie layer patch **511.** Unused portions **2623** may be stored by take up roll **2625.** In one example, the transfer cylinder **2621** may also act to laminate the flexible cover patch **517** to the tie layer patch 511 with the sensor device being at least partially received in the pocket **519.**

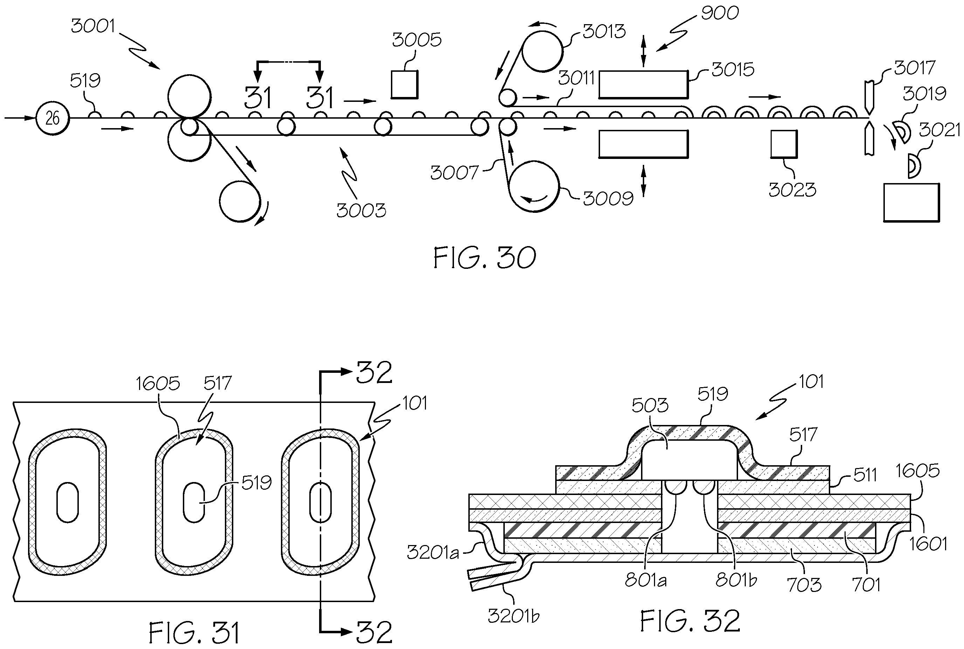

As further illustrated in **FIG. 26****,** the clear support substrate **2607** may be removed from the skin friendly adhesive layer **703** and stored in take-up roller **2624.** Final release liners (e.g., siliconized release liners) may then be added by release liner rolls **2626, 2627.** A folding device **2629** may be provided to provide a J-fold in one of portion of the release liner with the other release liner applied over the J-fold portion. As such, the release liner can comprise to portions **3201a**, **3201b** (see **FIG. 32**) to allow easy removal of the release liner when applying the wireless sensor patch to the skin surface **105** of the patient **103.**

**FIG. 30** is a continuation of **FIG. 26** illustrating further method steps in the method of manufacturing the plurality of wireless sensor patches **101**. A pair of die cut rollers **3001** operates to cut the flexible support sheet **1605,** the skin adhesive layer **1601** and the first and second portions **3201a, 3201b** to provide the final wireless sensor patch **101** illustrated in **FIGS. 31 and 32****.** Optionally, a conveyor **3003** may transport the wireless sensor patches **101** to be tested at testing station **3005.** The testing station **3005** may activate the wireless sensor patch (i.e., by depressing control button **510**) to determine proper functioning of the wireless sensor patch **101.** If proper function is not observed, the wireless sensor patch may be marked as defective for later removal from the final batch of wireless sensor patches **101**.

As further illustrated in **FIG. 30****,** the method may conclude with a packaging process. As shown, the conveyor can transport the wireless sensor patches **101** on a base substrate **3007** being unwound from a storage roll **3009.** Atop packaging sheet **3011** can likewise be unwound from a storage roll **3013.** Next, a sealing mechanism **3015** may periodically seal off a plurality of the wireless sensor patches **101** in a series of connected pouches that are subsequently separated from one another with a separation device **3017.** Once separated, the packaged devices **3019** can be stored in a storage bin 3021.

A printing device **3023** may be designed to print indicia including information relating to the wireless sensor patch **101** on the outside of the package. For example, the indicia **501** on the skin friendly adhesive patch **403** and/or the indicia **807** on the sensor device **503** may contain information that matches the information of indicia provided on a corresponding package housing each wireless sensor patch. Providing the information on the package can be helpful to determine information about the wireless sensor device contained within the package without having to open the package to retrieve the information from the indicia printed on the wireless sensor patch. Moreover, once the wireless sensor device is removed from the packaging, the information is not lost with the packaging since equivalent indicia is also provided on the wireless sensor patch **101.**

The method can also determine if any devices were marked as defective by the testing station **3005.** If so marked, the defective packaged devices **3019** may be removed prior after being separated and before entering the storage bin. For example, defective packaged devices **3019** may be stored within a defective storage bin (not shown) for subsequent processing or disposal. Still further, in some examples, the method can include the step of associating indicia of a portion of the wireless sensor patch (e.g., indicia **501** an d/or indicia **807**) with a batch of at least one source of assembly materials used to manufacture the wireless sensor patch. As such, one can easily cross reference material rolls with the particular wireless sensor devices including material from specific rolls. For example, the method can associate each storage roll of material with the particular wireless sensor patch produced. As an example, the information associated with the wireless sensor patch can be used to determine exactly which skin friendly membrane storage roll was used, and other rolls of materials. In such examples, wireless sensor patches may be quickly located that were made from various sources of assembly materials. Such information may be useful to recall certain wireless sensor patches that were made from a defective source of material.

The method disclosed herein provide multiple repeating steps positioned along an assembly path that may be performed periodically to sequentially produce the plurality of wireless sensor patches **101.** Manufacturing the sensor patches continuously from rolls of material can reduce assembly time and costs while providing consistently high quality wireless sensor patches.

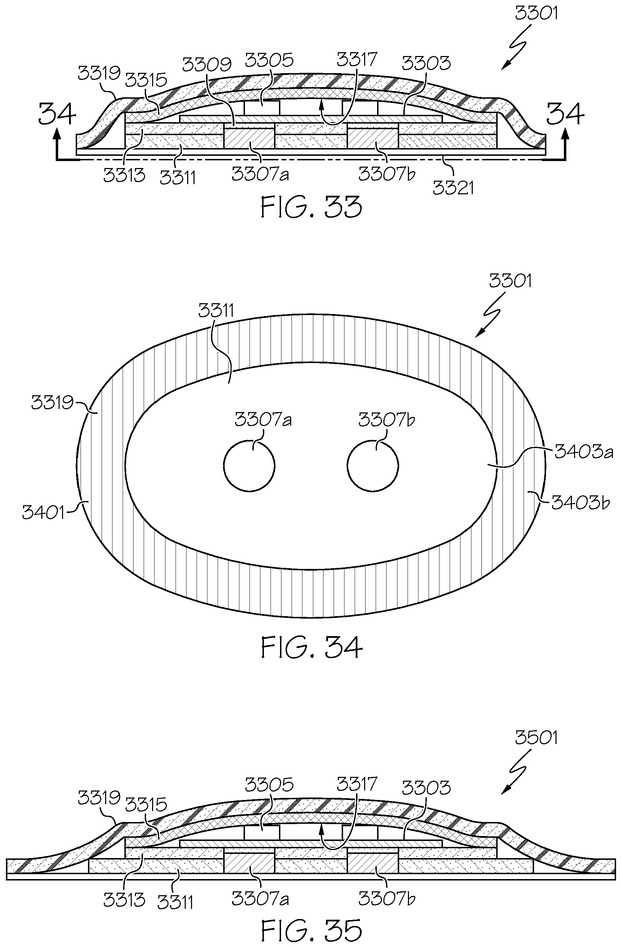

Methods of the disclosure can be modified to create a wide range of skin patches having similar, such as identical features to the wireless sensor patch **101** discussed above. **FIGS. 33-39** schematically illustrate various features of alternative wireless sensor patches that, like the wireless sensor patch **101,** include features that are not necessarily to scale for clarity. For instance, **FIG. 33** is cross-sectional view of another example wireless sensor patch **3301.** The wireless sensor patch **3301** can include a printed flexible circuit board **3303** that be provided with at least one electronic component, such as the illustrated plurality of various electronic components **3305** (e.g., silicon packaged electronic components), mounted to the printed flexible circuit board **3303.** The flexible circuit board **3303** is designed to allow a certain amount of flexing to permit partial or entire conformance of the printed flexible circuit board **3303** to the skin surface **105** of the patient **103.** As shown, the one or more electronic components **3305** extend from a first side of the printed flexible circuit board **3303** and may be selected in accordance with the desired characteristics of the sensor. For example, the electronic components **3305** may be provided to give the wireless sensor patch **3301** any one or combination of functional capabilities described with respect to the wireless sensor patch **101** above. In some examples, at least one electrode, such as the illustrated plurality of electrodes **3307a**, **3307b,** may be provided to extend from a second side of the printed flexible circuit board **3303.** Various electrode configurations may be provided such as an Ag-AgCI electrode with an optional electrolyte gel. The one or more electrodes may be placed in operable communication with an electrical trace or other portion of an electrical circuit supported by the substrate of the printed flexible circuit board **3303.** For example, a conductive coupling element **3309** may be used to fix the electrodes **3307a**, **3307b** to the appropriate location of the printed flexible circuit board **3303.** In some examples, the conductive coupling element 3309 may comprise a conductive pressure sensitive adhesive and/or gel.

A skin-friendly adhesive patch **3311,** similar or identical in composition to the skin-friendly adhesive patch **403** of the wireless sensor patch **3301** discussed above, may be mounted with respect to a second side of the printed flexible circuit board **3303.** For example, as shown, a tie layer **3313** may mount the skin-friendly adhesive patch **3311** to the second side of the printed flexible circuit board **3303.** The tie layer **3313** may comprise a double-coated breathable material or may simply comprise a double-sided adhesive tape.

In one example, the skin-friendly adhesive patch **3311** may comprise a hydrocolloid adhesive patch although other skin-friendly adhesives may be provided such as integrated hydrocolloid or other adhesives capable of absorbing moisture. For example, the skin-friendly adhesive patch **3311** can comprise a hydrocolloid such as the hydrocolloid material disclosed in any one of U.S. Patent No 7,335,416 that issued on February 6, 2008, U.S. Patent No 6,710,100 that issued on March 23, 2004, U.S. Patent No 6,583,220 that issued on June 24, 2003, U.S. Patent No 6,326,421 that issued on December 4, 2001, U.S. Patent Application No. 12/866,750 filed August 9, 2010, and U.S. Provisional Patent 61/467,553 filed March 25, 2011.

Any of the wireless sensor skin patches discussed in the invention disclosure may include a cushion layer for patient comfort and/or to help protect the printed flexible circuit board (if provided) and/or protect other electrical components such as electrical components associated with the printed flexible circuit board. For example, as shown in **FIG. 33****,** the wireless sensor patch **3301** can further include a cushion layer **3315** that may help protect the printed flexible circuit board **3303** and/or associated electronic components **3305** from damage. Indeed, the cushion layer **3315** may protect the circuit board and/or electronic components from externally applied forced (e.g., impact forces) that may otherwise damage the wireless sensor patch **3301.** Moreover, the cushion layer **3315** may also enhance comfort to the patient wearing the patch or other individuals encountering the wireless sensor patch **3301.** Indeed, rather than encounter relatively rigid electronic components **3305,** the cushion layer **3315** may act as a protective bumper to avoid otherwise unpleasant encounters with the relatively rigid electronic components. In one example, the cushion layer **3315** may comprise a fabric represented by the cross-hatch pattern illustrated in the drawings. The illustrated fabric of the cushion layer **3315** comprises a nonwoven fabric although woven fabrics may be provided in further examples. The cushion layer **3315** may also include an adhesive applied to a surface **3317** facing the electronic components and printed circuit board. For example, the surface **3317** may be provided with a pressure sensitive adhesive that may facilitate mounting of the cushion layer **3315** in place by adhesive mounting to the electronic components **3305,** printed flexible circuit board **3303** and/or tie layer **3313.**

The wireless sensor patch **3301** can further include a flexible cover patch **3319** mounted with respect to the skin-friendly adhesive patch **3311** with the electronic component **3305** positioned within a space between the flexible cover patch **3319** and the skin-friendly adhesive patch **3311.** As shown, the flexible cover patch **3319** can be positioned over the cushion layer **3315** such that the cushion layer **3315** is positioned to extend between the electronic component **3305** and the flexible cover patch **3319.** The flexible cover patch **3319** may comprise a polymeric member, such as a closed cell foam material that may be substantially water resistant yet breathable to help protect the electrical components **3305** and the printed flexible circuit board **3303.**

**FIG. 34** is a bottom view of the wireless sensor patch **3301** wherein a release liner **3321,** designed to preserve the adhesive surfaces from contamination prior to application, is not illustrated for clarity. As represented by the vertical lines set forth in **FIG. 34****,** the flexible cover patch **3319** may also include an adhesive layer **3401** designed to mount the flexible cover patch **3319** to the cushion layer **3315.** The adhesive layer **3401** can comprise a pressure sensitive adhesive such as rubber-based adhesive, acrylic adhesive or silicone adhesive that allows a peripheral adhesive portion **3403b** defined by an adhesive layer **3401** of the flexible cover patch **3319** to immediately adhere to the skin surface **105** upon application of the wireless sensor patch **3301.** Moreover, as mentioned previously, the wireless sensor patch **3301** can comprise the skin-friendly adhesive patch **3311.**

As shown in **FIG. 34****,** the peripheral adhesive portion **3403b** at an outer periphery of the adhesive layer **3401** can circumscribe a skin-friendly portion **3403a** of the skin-friendly adhesive patch **3311.** Indeed, as apparent in **FIGS. 33-39****,** the flexible cover patch of the wireless skin patch can have a footprint that is larger than a foot print of the skin-friendly adhesive patch. As such, each wireless skin patch of the present disclosure can include an adhesive footprint with a skin-friendly adhesive portion defined by the skin-friendly adhesive patch that is optionally circumscribed by a peripheral adhesive portion defined by an adhesive layer of the flexible cover patch. For instance, by way of illustration, **FIG. 34** illustrates an adhesive footprint of the wireless skin patch **3301** with the skin-friendly portion **3403a** defined by the skin-friendly adhesive patch **3311** that is circumscribed by the peripheral adhesive portion **3403b** defined by an adhesive layer **3401** of the flexible cover patch **3319.**

As such, an outer peripheral adherence of the wireless sensor patch **3301** to the skin surface **105** of the patient **103** may be achieved. At the same time, the skin-friendly adhesive patch **3311** may be held in place against the skin surface **105** to allow sufficient time for the skin-friendly adhesive patch **3311** to cure into an effective adhesive member. The skin-friendly adhesive patch **3311** allows the wireless sensor patch **3301** to be applied to the skin surface for a significant length of time without aggravating the skin surface when compared to the adhesive layer **3401.** At the same time, a relatively small peripheral portion **3403b** of the adhesive layer **3401** may allow the peripheral portions of the patch to be immediately adhered to the skin surface while allowing the skin-friendly portion **3403a** of the skin-friendly adhesive patch **3311** sufficient time to cure.

**FIG. 35** is cross-sectional view of still another example wireless sensor patch **3501** in accordance with additional aspects of the disclosure. Unless otherwise indicated, the wireless sensor patch **3501** can include identical or similar features discussed with respect to the wireless sensor patch **3301** discussed above and referenced in **FIGS. 33-34****.** Referring to **FIGS. 33-35****,** the footprint of the wireless sensor patch **3501** shown in **FIG. 35** is larger than the footprint of the wireless sensor patch **3301** illustrated in **FIGS. 33-34****.** In such applications, the footprints of the skin-friendly adhesive patch **3311** and/or flexible cover patch **3319** of the wireless sensor patch **3501** can likewise be greater than the corresponding footprints of the skin-friendly adhesive patch and/or flexible cover patch of the wireless sensor patch **3301.** As illustrated, the footprint of the skin-friendly adhesive patch can be increased with an increase in the overall footprint of the wireless sensor patch to provide a more skin-friendly adhesive patch that exposes a greater portion of the skin to the skin-friendly adhesive.

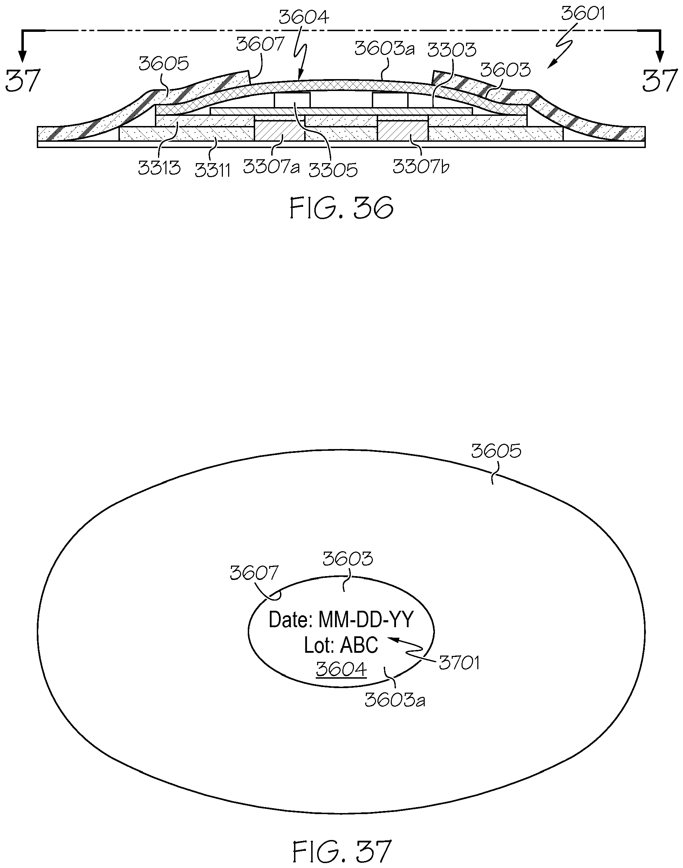

**FIG. 36** is cross-sectional view of yet another example wireless sensor patch **3601** in accordance with aspects of the disclosure. Unless otherwise indicated, the wireless sensor patch **3601** can include identical or similar features discussed with respect to the wireless sensor patches **3301, 3501** discussed above and referenced in **FIGS. 33-35****.** However, as shown, the cushion layer **3603** of the wireless sensor patch **3601** may comprise a foam cushion material or other material that will allow indicia **3701** (see **FIG. 37**) to be printed on an outer surface **3604** of the cushion layer **3603.** In such examples, as shown in **FIGS. 36 and 37**, the flexible cover patch **3605** may define a viewing port **3607** configured to permit viewing of a portion **3603a** the cushion layer **3603** through the viewing port **3607** of the flexible cover patch **3605.** In some examples, indicia **3701,** such as identifying information, may be printed on the portion **3603a** of the cushion layer **3603** and viewed through the viewing port **3607** defined by the flexible cover patch **3605.** In some examples, the viewing port **3607** may comprise an aperture although a translucent or transparent membrane or other port allowing viewing may be provided in further examples. The flexible cover patch of any of the example wireless sensor patches of the disclosure may include a view port to allow viewing of indicia underlying the flexible cover patch. For example, any of the flexible cover patches of any of the example wireless sensor patches may include a view port comprising an aperture allowing viewing of indicia printed on a surface (e.g., a surface of a cushion layer) that would otherwise be obscured by the flexible cover patch.

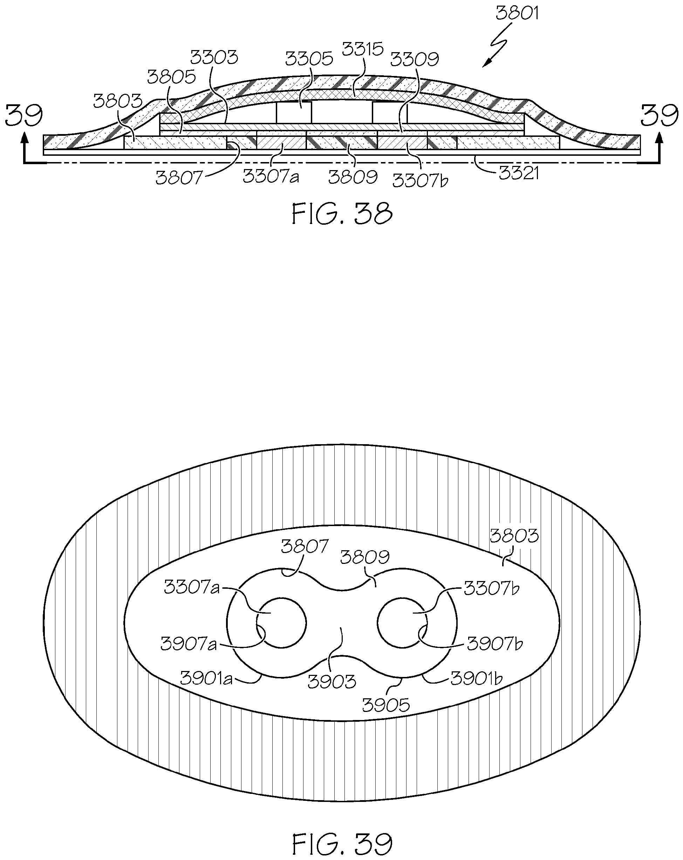

**FIG. 38** is cross-sectional view of yet another example wireless sensor patch **3801.** The wireless sensor patch **3801** can include a printed flexible circuit board **3303** that be provided with various previous-described electronic components **3305.** At least one electrode, such as the illustrated plurality of electrodes **3307a, 3307b** may extend from the second side of the printed flexible circuit board **3303.** As mentioned previously, the electrodes may comprise Ag-AgCI electrodes that may optionally include electrolyte gel and may be placed in operable communication with an electrical trace or other portion of an electrical circuit supported by the substrate of the printed flexible circuit board **3303.** For example, a conductive coupling element **3309** may be used to fix the electrodes **3307a**, **3307b** to the appropriate location of the printed flexible circuit board **3303.** For instance, the conductive coupling element **3309** may comprise a conductive pressure sensitive adhesive and/or gel. A skin-friendly adhesive patch **3803,** similar or identical in composition to the skin-friendly adhesive patches **403, 3311** discussed above, may be mounted with respect to the printed circuit board **3303** by way of a tie layer **3805.** The tie layer **3805** may comprise the illustrated double-sided adhesive tape although other tie layers (e.g., the tie layer **3313** discussed above) may be used in further examples.

In any of the example wireless skin patched described through the disclosure, any of the wireless skin patches including one or more electrodes (e.g., electrodes **3307a, 3307b**) may include an optional electrode insulation member. For example, as illustrated in **FIGS. 38 and 39****,** an electrode insulation member **3809** may be positioned within at least one through aperture **3807.** Although a single aperture **3807** is shown, a plurality of apertures **3807** may be provided in further examples wherein at least one of the apertures includes an electrode insulation member **3809.** The electrode insulation member **3809** can circumscribe at least one electrode to prevent contact between the circumscribed electrode and the skin-friendly adhesive patch **3803.** For example, as shown, the electrode insulation member **3809** may circumscribe both of the electrodes **3307a**, **3307b** although the electrode insulation member **3809** may circumscribe a single electrode or less than all of a plurality of electrodes in further examples. In some examples, the electrode insulation member **3809** is designed to electrically insulate the electrodes **3307a, 3307b** from one another or other components. In one example, the electrode insulation member can comprise a closed-cell foam, nonwoven fabric or other material configured to electrically insulate one or more of the electrodes. Electrical insulation can be particularly beneficial since the skin-friendly adhesive patch **3803** will tend absorb significant levels of liquid (e.g., water) from the skin surface **105** of a patient **103** over time. The electrode insulation member **3809** can prevent direct exposure of the electrodes **3307** to the skin-friendly adhesive patch **3803,** thereby avoiding undesired electrical communication (e.g., short circuiting) of the electrodes **3307a, 3307b** that may otherwise occur through the saturated skin-friendly adhesive patch **3803.**

In one example, the electrodes may extend through an aperture of the electrode insulation member **3809.** For instance, as shown in **FIG. 39****,** the first electrode **3307a** may extend through a first aperture **3907a** of the electrode insulation member **3809** while the second electrode **3307b** may extend through the second aperture **3907b**. As such, in some examples each electrode **3307a, 3307b** can extend through a single through aperture **3807** of the skin-friendly adhesive patch **3803** while extending through corresponding apertures **3907a**, **3907b** of the electrode insulation member **3809.** In such an example, the electrode insulation member **3809** can therefore prevent both the first electrode **3307a** and the second electrode **3307b** from contacting the skin-friendly adhesive patch **3803.** Insulating both electrodes can help further electrically insulate the electrodes from one another and/or from other components. Although not shown, the electrode insulation member may be provided to insulate a single electrode. Insulating a single electrode may reduce material costs, increase available skin-friendly adhesive surface area contact with the skin while still electrically insulating the insulated electrode from the remaining electrode(s).

As further shown in **FIG. 39****,** the electrode insulation member **3809** can optionally comprise bulbous portions **3901a**, **3901b** having a footprint larger than the corresponding footprint of the electrodes **3307a**, **3307b** to allow the electrode insulating member to circumscribe each of the electrodes. Moreover, a reduced neck portion **3903** may be provided between the bulbous portions **3901a, 390b** such that the outer periphery **3905** of the electrode insulation member **3809** together with the apertures **3907** provides the electrode insulation member **3809** as an 8-shaped electrode insulation member having a shape of the number "8". Providing the reduced neck portion **3903** allows the bulbous portions to be connected together while still maximizing the surface area of the skin-friendly adhesive available for contacting the skin surface. Still further, the reduced neck portion can help increase the electrical insulation properties in the area positioned between the electrodes, thereby enhancing thermal insulation characteristics while maximizing the surface area of the skin-friendly adhesive available for contacting the skin surface. Although not shown, the bulbous portions **3901** may simply comprise donuts that are not connected to one another. In such an example, a single donut may be provided about a single electrode to electrically insulate a selected electrode from the other electrode(s) and/or other components. Alternatively, a plurality of donuts may be provided, for example, about each electrode to electrically insulate all of the electrodes from one another and/or other components.

## Claims

1. A method of manufacturing a plurality of wireless sensor patches comprising the steps of:
(I) unwinding a skin-friendly adhesive membrane (901) from a skin-friendly adhesive membrane storage roll (903), wherein the skin-friendly adhesive membrane includes a substrate sheet (905) carrying a layer of skin-friendly adhesive (907) and a first release liner (909) carried by the layer of skin-friendly adhesive, wherein the layer of skin-friendly adhesive is sandwiched between the first release liner and the substrate sheet;
(II) kiss cutting the substrate sheet and the skin-friendly adhesive to the first release liner to define a skin-friendly adhesive patch;
(III) unwinding a flexible support membrane (1501) from a support membrane storage roll (1503), wherein the flexible support membrane includes a flexible support sheet (1605) with a skin adhesive layer (1601) applied to a first face (1603) of the flexible support sheet and a second release liner (1607) carried by the skin adhesive layer with the skin adhesive layer being sandwiched between the second release liner and the flexible support sheet;
(IV) kiss cutting the flexible support sheet and the skin adhesive layer to the second release liner to define an opening (1901) extending through the flexible support sheet and the skin adhesive layer;
(V) removing the second release liner to expose the skin adhesive layer;
(VI) laminating the substrate sheet to the flexible support sheet with the skin adhesive layer of the flexible support membrane;
(VII) unwinding a tie layer membrane (1513) from a tie layer membrane storage roll (1515), wherein the tie layer membrane includes a tie layer (2101) carrying a third release liner (2103);
(VIII) kiss cutting the tie layer to the third release liner to define a tie layer patch (511);
(IX) laminating the tie layer patch to the flexible support sheet;
(X) cutting at least one probe opening through the tie layer membrane in alignment with the opening defined during step (IV);
(XI) removing the third release liner to expose the tie layer patch laminated to the flexible support sheet;
(XII) mounting a sensor device (503) with respect to the flexible support membrane with a sensor probe in alignment with corresponding openings defined during steps (IV) and (X);
(XIII) unwinding a flexible cover membrane (2613) from a flexible cover membrane storage roll (2615);
(XIV) forming a pocket (519) within the flexible cover membrane;
(XV) cutting the flexible cover membrane to define a flexible cover patch including the pocket;
(XVI) laminating the flexible cover patch to the tie layer patch with the sensor device being at least partially received in the pocket; and
(XVII) cutting the flexible support sheet and the skin adhesive layer to provide a wireless sensor patch (101).

2. The method of claim 1, wherein step (XVII) provides an outer periphery of the skin adhesive layer circumscribing the skin-friendly adhesive patch.

3. The method of any one of claims 1 and 2, wherein the skin-friendly adhesive (907) comprises a hydrocolloid skin adhesive.

4. The method of any one of claims 1-3, wherein the flexible support membrane (1501) comprises a fabric.

5. The method of claim4, wherein the fabric comprises a nonwoven fabric.

6. The method of any one of claims 1-5, wherein step (XVII) is performed periodically to sequentially produce a plurality of wireless sensor patches (101).

7. The method of any one of claims 1-6, further comprising the step of providing indicia to a portion of each wireless sensor patch (101) containing information that matches information of indicia provided on a corresponding package housing each wireless sensor patch.

8. The method of any one of claims 1-7, further comprising the step of associating indicia of a portion of the wireless sensor patch (101) with a batch of at least one source of assembly materials used to manufacture the wireless sensor patch.

## Patentansprüche

1. Verfahren zur Herstellung einer Vielzahl von Pflastern mit drahtlosen Sensoren, das die folgenden Schritte umfasst:
(I) Abwickeln einer hautfreundlichen Klebstoffmembran (901) von einer Lagerrolle (903) der hautfreundlichen Klebstoffmembran, wobei die hautfreundliche Klebstoffmembran eine Substratlage (905), die eine Schicht aus hautfreundlichem Klebstoff (907) trägt, und einen ersten Trennüberzug (909), der von der Schicht aus hautfreundlichem Klebstoff getragen wird, umfasst, wobei die Schicht aus hautfreundlichem Klebstoff zwischen dem ersten Trennüberzug und der Substratlage eingeschoben ist;
(II) Anstanzen der Substratlage und des hautfreundlichen Klebstoffs zu dem ersten Trennüberzug, um ein Pflaster mit hautfreundlichem Klebstoff zu definieren;
(III) Abwickeln einer flexiblen Trägermembran (1501) von einer Lagerrolle (1503) der Trägermembran, wobei die flexible Trägermembran eine flexible Trägerlage (1605) mit einer Hautklebstoffschicht (1601), die auf eine erste Fläche (1603) der flexiblen Trägerlage aufgebracht wird, und einen zweiten Trennüberzug (1607), der von der Hautklebstoffschicht getragen wird, umfasst, wobei die Hautklebstoffschicht zwischen dem zweiten Trennüberzug und der flexiblen Trägerlage eingeschoben ist;
(IV) Anstanzen der flexiblen Trägerlage und der Hautklebstoffschicht zu dem zweiten Trennüberzug, um eine Öffnung (1901) zu definieren, die sich durch die flexible Trägerschicht und die Hautklebstoffschicht erstreckt;
(V) Entfernen des zweiten Trennüberzugs, um die Hautklebstoffschicht freizulegen;
(VI) Laminieren der Substratlage an die flexible Trägerschicht mit der Hautklebstoffschicht der flexiblen Trägermembran;
(VII) Abwickeln einer Haftvermittlerschichtmembran (1513) von einer Lagerrolle (1515) der Haftvermittlerschichtmembran, wobei die Haftvermittlerschichtmembran eine Haftvermittlerschicht (2101) umfasst, die einen dritten Trennüberzug (2103) trägt;
(VIII) Anstanzen der Haftvermittlerschicht zu dem dritten Trennüberzug, um ein Haftvermittlerschicht-Pflaster (511) zu definieren;
(IX) Laminieren des Haftvermittlerschicht-Pflasters an die flexible Trägerlage;
(X) Schneiden wenigstens einer Sondenöffnung durch die Haftvermittlerschichtmembran in Ausrichtung mit der während des Schritts (IV) definierten Öffnung;
(XI) Entfernen des dritten Trennüberzugs, um das Haftvermittlerschicht-Pflaster, das an die flexible Trägerschicht laminiert ist, freizulegen;
(XII) Montieren einer Sensorvorrichtung (503) in Bezug auf die flexible Trägermembran mit einer Sensorsonde in Ausrichtung mit entsprechenden Öffnungen, die während der Schritte (IV) und (X) definiert wurden;
(XIII) Abwickeln einer flexiblen Deckmembran (2613) von einer Lagerrolle (2615) für die flexible Deckmembran;
(XIV) Ausbilden einer Tasche (519) in der flexiblen Deckmembran;
(XV) Schneiden der flexiblen Deckmembran, um ein flexibles Deckpflaster, das die Tasche enthält, zu definieren;
(XVI) Laminieren des flexiblen Deckpflasters an das Haftvermittlerschicht-Pflaster, wobei die Sensorvorrichtung wenigstens teilweise in der Tasche aufgenommen ist; und
(XVII) Schneiden der flexiblen Trägerlage und der Hautklebstoffschicht, um ein Pflaster (101) mit drahtlosem Sensor bereitzustellen.

2. Verfahren nach Anspruch 1, wobei der Schritt (XVII) einen Außenumfang der Hautklebstoffschicht bereitstellt, der das Pflaster mit hautfreundlichem Klebstoff abgrenzt.

3. Verfahren nach einem der Ansprüche 1 und 2, wobei der hautfreundliche Klebstoff (907) einen hydrokolloiden Hautklebstoff umfasst.

4. Verfahren nach einem der Ansprüche 1 - 3, wobei die flexible Trägermembran (1501) ein Gewebe umfasst.

5. Verfahren nach Anspruch 4, wobei das Gewebe ein nicht gewebtes bzw. Vliesgewebe umfasst.

6. Verfahren nach einem der Ansprüche 1 - 5, wobei der Schritt (XVII) regelmäßig durchgeführt wird, um nacheinander eine Vielzahl von Pflastern (101) mit drahtlosen Sensoren herzustellen.

7. Verfahren nach einem der Ansprüche 1 - 6, das ferner den Schritt der Bereitstellung von Markierungen an einem Abschnitt jedes Pflasters (101) mit drahtlosen Sensoren umfasst, welche Informationen enthalten, die zu Informationen von Markierungen passen, die auf einer entsprechenden Verpackung bereitgestellt sind, welche jedes Pflaster mit drahtlosem Sensor aufnimmt.

8. Verfahren nach einem der Ansprüche 1 - 7, das ferner den Schritt des Zuordnens von Markierungen eines Abschnitts des Pflasters (101) mit drahtlosem Sensor zu einem Los wenigstens einer Quelle von Montagematerialien, die verwendet werden, um das Pflaster mit drahtlosem Sensor herzustellen, umfasst.

## Revendications

1. Procédé de fabrication d'une pluralité de timbres capteurs sans fil, comportant les étapes de :
(I) déroulage d'une membrane adhésive douce pour la peau (901) à partir d'un rouleau de stockage (903) de membrane adhésive douce pour la peau, dans lequel la membrane adhésive douce pour la peau comprend une feuille de substrat (905) portant une couche d'adhésif doux pour la peau (907) et une première protection antiadhésive (909) portée par la couche d'adhésif doux pour la peau, dans lequel la couche d'adhésif doux pour la peau est enserrée entre la première protection antiadhésive et la feuille de substrat ;
(II) découpe à mi-chair de la feuille de substrat et de l'adhésif doux pour la peau jusqu'à la première protection antiadhésive afin de définir un timbre adhésif doux pour la peau ;
(III) déroulage d'une membrane de support flexible (1501) à partir d'un rouleau de stockage (1503) de membrane de support, dans lequel la membrane de support flexible comprend une feuille de support flexible (1605) avec une couche d'adhésif pour la peau (1601) appliquée sur une première face (1603) de la feuille de support flexible et une deuxième protection antiadhésive (1607) portée par la couche d'adhésif pour la peau, la couche d'adhésif pour la peau étant enserrée entre la deuxième protection antiadhésive et la feuille de support flexible ;
(IV) découpe à mi-chair de la feuille de support flexible et de la couche d'adhésif pour la peau jusqu'à la deuxième protection antiadhésive afin de définir une ouverture (1901) s'étendant à travers la feuille de support flexible et la couche d'adhésif pour la peau ;
(V) retrait de la deuxième protection antiadhésive afin d'exposer la couche d'adhésif pour la peau ;
(VI) stratification de la feuille de substrat sur la feuille de support flexible avec la couche d'adhésif pour la peau de la membrane de support flexible ;
(VII) déroulage d'une membrane de couche de liaison (1513) à partir d'un rouleau de stockage (1515) de membrane de couche de liaison, dans lequel la membrane de couche de liaison comprend une couche de liaison (2101) portant une troisième protection antiadhésive (2103) ;
(VIII) découpe à mi-chair de la couche de liaison jusqu'à la troisième protection antiadhésive afin de définir un timbre de couche de liaison (511) ;
(IX) stratification du timbre de couche de liaison sur la feuille de support flexible ;
(X) découpe d'au moins une ouverture de sonde à travers la membrane de couche de liaison, en alignement avec l'ouverture définie au cours de l'étape (IV) ;
(XI) retrait de la troisième protection antiadhésive afin d'exposer le timbre de couche de liaison stratifié sur la feuille de support flexible ;
(XII) montage d'un dispositif de capteur (503) par rapport à la membrane de support flexible avec une sonde de capteur alignée sur les ouvertures correspondantes définies au cours des étapes (IV) et (X) ;
(XIII) déroulage d'une membrane de recouvrement flexible (2613) à partir d'un rouleau de stockage (2615) de membrane de recouvrement flexible ;
(XIV) formation d'une poche (519) au sein de la membrane de recouvrement flexible ;
(XV) découpe de la membrane de recouvrement flexible afin de définir un timbre de recouvrement flexible comprenant la poche ;
(XVI) stratification du timbre de recouvrement flexible sur le timbre de couche de liaison, le dispositif de capteur étant au moins partiellement logé dans la poche ; et
(XVII) découpe de la feuille de support flexible et de la couche d'adhésif pour la peau afin de fournir un timbre capteur sans fil (101).

2. Procédé selon la revendication 1, dans lequel l'étape (XVII) prévoit une périphérie extérieure de la couche d'adhésif pour la peau délimitant le timbre adhésif doux pour la peau.

3. Procédé selon l'une quelconque des revendications 1 et 2, dans lequel l'adhésif doux pour la peau (907) comporte un adhésif pour la peau hydrocolloïde.

4. Procédé selon l'une quelconque des revendications 1 à 3, dans lequel la membrane de support flexible (1501) comporte un tissu.

5. Procédé selon la revendication 4, dans lequel le tissu comporte un tissu non-tissé.

6. Procédé selon l'une quelconque des revendications 1 à 5, dans lequel l'étape (XVII) est réalisée périodiquement afin de produire de façon séquentielle une pluralité de timbres capteurs sans fil (101).

7. Procédé selon l'une quelconque des revendications 1 à 6, comportant en outre l'étape de fourniture de vignettes sur une portion de chaque timbre capteur sans fil (101), lesquelles contiennent des informations qui correspondent aux informations des vignettes prévues sur un emballage correspondant recevant chaque timbre capteur sans fil.

8. Procédé selon l'une quelconque des revendications 1 à 7, comportant en outre l'étape d'association des vignettes d'une portion du timbre capteur sans fil (101) à un lot d'au moins une source de matériaux d'assemblage utilisés pour fabriquer le timbre capteur sans fil.
